(19)

Europäisches
Patentamt
European
Patent Office
Office européen
des brevets

(11) **EP 1 696 734 B1**

(12) **EUROPEAN PATENT SPECIFICATION**

(45) Date of publication and mention
of the grant of the patent:
**15.08.2012 Bulletin 2012/33**

(51) Int Cl.:
*A23K 1/16* (2006.01)    *A23K 1/18* (2006.01)
*A61P 1/00* (2006.01)

(21) Application number: **04812573.6**

(86) International application number:
**PCT/US2004/040086**

(22) Date of filing: **01.12.2004**

(87) International publication number:
**WO 2005/053426 (16.06.2005 Gazette 2005/24)**

(54) **METHODS AND KITS RELATED TO ADMINISTRATION OF A FRUCTOOLIGOSACCHARIDE**

VERFAHREN UND KITS FÜR DIE VERARBEITUNG EINES FRUCTOOLIGOSACCHARIDS

PROCEDES ET NECESSAIRES SE RAPPORTANT A L'ADMINISTRATION D'UN FRUCTO-OLIGO-SACCHARIDE

(84) Designated Contracting States:
**AT BE BG CH CY CZ DE DK EE ES FI FR GB GR HU IE IS IT LI LT LU MC NL PL PT RO SE SI SK TR**

(30) Priority: **01.12.2003 US 724839**

(43) Date of publication of application:
**06.09.2006 Bulletin 2006/36**

(73) Proprietor: **THE IAMS COMPANY**
**Dayton, Ohio 45414-5801 (US)**

(72) Inventors:
• **SUNVOLD, Gregory, Dean**
**Lewisburg, OH 45338 (US)**
• **BOILEAU, Thomas, William-Maxwell**
**Springfield, OH 45504 (US)**
• **VICKERS, Robert, Jason**
**Jackson, TN 38305 (US)**

(74) Representative: **Goodier, Claire-Louise et al**
**N.V.Procter & Gamble Services Company S.A.**
**Temselaan 100**
**1853 Strombeek-Bever (BE)**

(56) References cited:
**EP-A- 1 125 507      WO-A-99/22604**
**US-A- 5 776 524      US-A- 5 952 033**
**US-B1- 6 596 332**

• **HUSSEIN, H.S., FLICKINGER, E.A., AND FAHEY, G.C.: "Petfood applications of inulin and oligofructose" JOURNAL OF NUTRITION., vol. 129, 1999, pages 1454-1456, XP002323326 USWISTAR INSTITUTE OF ANATOMY AND BIOLOGY, PHILADELPHIA, PA,**
• **WILLARD M D ET AL: "EFFECTS OF DIETARY SUPPLEMENTATION OF FRUCTO-OLIGOSACCHARIDES ON SMALL INTESTINAL BACTERIAL OVERGROWTH IN DOGS" AMERICAN JOURNAL OF VETERINARY RESEARCH, vol. 55, no. 5, 1 May 1994 (1994-05-01), pages 654-659, XP000645762 ISSN: 0002-9645**
• **DATABASE BIOSIS [Online] BIOSCIENCES INFORMATION SERVICE, PHILADELPHIA, PA, US; March 2003 (2003-03), GRIESHOP CHRISTINE M ET AL: "Gastrointestinal and immunological responses of senior dogs to chicory and mannanoligosaccharides." XP002324194 Database accession no. PREV200300381566 & FASEB JOURNAL, vol. 17, no. 4-5, March 2003 (2003-03), pages Abstract No. 202.5 URL-http://ww, FASEB MEETING ON EXPERIMENTAL BIOLOGY: TRANSLATING THE GENOME; SAN DIEGO, CA, USA; APRIL 11-15, 2003 ISSN: 0892-6638**

EP 1 696 734 B1

**(Cont. next page)**

- DATABASE BIOSIS [Online] BIOSCIENCES INFORMATION SERVICE, PHILADELPHIA, PA, US; 20 March 2002 (2002-03-20), PATIL AVINASH R ET AL: "Supplementation of soluble fiber to wet cat food affects fecal microflora of cats" XP002324182 Database accession no. PREV200200396597 & FASEB JOURNAL, vol. 16, no. 4, 20 March 2002 (2002-03-20), page A654, ANNUAL MEETING OF THE PROFESSIONAL RESEARCH SCIENTISTS ON EXPERIMENTAL BIOLOGY; NEW ORLEANS, LOUISIANA, USA; APRIL 20-24, 2002 ISSN: 0892-6638
- ZENTEK, J., MARQUART, B., PIETRZAK, T., BALLÈVRE, O., AND ROCHAT, F.: "Dietary effects on bifidobacteria and clostridium perfringens in the canine intestinal tract" JOURNAL OF ANIMAL PHYSIOLOGY AND ANIMAL NUTRITION., vol. 87, November 2003 (2003-11), pages 397-407, XP002324180 DEBLACKWELL, BERLIN.
- DATABASE BIOSIS [Online] BIOSCIENCES INFORMATION SERVICE, PHILADELPHIA, PA, US; 1996, OHTA ATSUTANE ET AL: "Prevention of coprophagy modifies magnesium absorption in rats fed with fructo-oligosaccharides" XP002324183 Database accession no. PREV199699081521 & BRITISH JOURNAL OF NUTRITION, vol. 75, no. 5, 1996, pages 775-784, ISSN: 0007-1145
- OHTA ATSUTANE ET AL: "Prevention of coprophagy modifies magnesium absorption in rats fed with fructo-oligosaccharides", BIOSIS, XP002324183,
- DIEZ, M., ET AL.: "THE INFLUENCE OF SUGAR BEET FIBER, GUAR GUM AND INULIN ON NUTRIENT DIGESTIBILITIES. WATER CONSUMPTION AND PLASMA METABOLITES IN HEALTHY BEAGLE DOGS", RESEARCH IN VETERINARY SCIENCE., vol. 64, 1998, pages 91-96, GBBRITISH VETERINARY ASSOCIATION, LONDON. ISSN: 0034-5288
- FLICKINGER, E.A., ET AL: "NUTRIENT DIGESTIBILITIES, MICROBIAL POPULATIONS AND PROTEIN CATABOLITES AS AFFECTED BY FRUCTAN SUPPLEMENTATION OF DOG DIETS", JOURNAL OF ANIMAL SCIENCE, vol. 81, August 2003 (2003-08), pages 2008-2018, USNEW YORK, NY ISSN: 0021-8812
- TWOMEY, L.N., ET AL.: "THE EFFECTS OF ADDED FRUCTOOLIGOSACCHARIDE (RAFTILOSE P95) AND INULINASE ON FAECAL QUALITY AND DIGESTIBILITY IN DOGS", ANIMAL FEED SCIENCE AND TECHNOLOGY, vol. 108, 25 August 2003 (2003-08-25), pages 83-93, Elsevier ISSN: 0377-8401
- DATABASE BIOSIS [Online] BIOSCIENCES INFORMATION SERVICE, PHILADELPHIA, PA, US; 20 March 2002 (2002-03-20), CZARNECKI-MAULDEN GAIL L ET AL: "Chicory root, a source of soluble fiber, increases apparent calcium digestibility in dogs", Database accession no. PREV200200396596
- CZARNECKI-MAULDEN GAIL L ET AL: "Chicory root, a source of soluble fiber, increases apparent calcium digestibility in dogs", FASEB JOURNAL, vol. 16, no. 4, 20 March 2002 (2002-03-20) , page A654, & ANNUAL MEETING OF THE PROFESSIONAL RESEARCH SCIENTISTS ON EXPERIMENTAL BIOLOGY; NEW ORLEANS, LOUISIANA, USA; APRIL 20-24, 2002 ISSN: 0892-6638
- CZARNECKI-MAULDEN GAIL L ET AL: "Chicory root, a source of soluble fiber, increases apparent calcium digestibility in dogs", 20 March 2002 (2002-03-20), FASEB JOURNAL, VOL. 16, NR. 4, PAGE(S) A654, ANNUAL MEETING OF THE PROFESSIONAL RESEARCH SCIENTISTS ON EXPERIMENTAL BIOLOGY; NEW ORLEANS, LOUISIANA, USA; APRIL 20-24, 2002 ISSN: 0892-6638
- HOWARD, M.D., ET AL: "Source of dietary fiber fed to dogs affects nitrogen and energy metabolism and intestinal microflora populations", NUTRITION RESEARCH., vol. 20, no. 10, 2000, pages 1473-1484, ISSN: 0271-5317
- OHTA ATSUTANE ET AL: "Prevention of coprophagy modifies magnesium absorption in rats fed with fructo-oligosaccharides", BIOSIS, XP002324183,

**Description**

FIELD OF THE INVENTION

The present invention is directed to methods of using companion animal compositions comprising fructooligosaccharide, as well as kits comprising such compositions.

BACKGROUND OF THE INVENTION

The anatomy, physiology, and microbial ecology of the mammalian gastrointestinal tract differs among humans, dogs, cats, and other species. For example, when normalized to body length or weight, total length of the canine and feline gastrointestinal tract is shorter than that of omnivorous mammals such as humans, and because of transit time differences and dietary patterns, the bacterial flora of the intestine also varies. Since the bacterial flora of the intestine is primary responsible for the breakdown of fermentable fibers such as fructooligosaccharide, it is expected that different species will uniquely utilize fermentable fibers.

Few studies have directly compared the ability of dogs, cats and humans to utilize dietary fibers. One study has compared the ability of the bacterial flora of dog, cat and human large intestine to metabolize several dietary fibers. Butyrate production from the tested fermentable fibers, which is characteristic of fiber metabolism, was about 50% greater for the human samples compared to either cat or dog and lactate production was about 73% lower for cat and human compared to dog samples. See Sunvold et al., "In vitro fermentation of cellulose, beet pulp, citrus pulp, and citrus pectin using fecal inoculum from cats, dogs, horses, and pigs and ruminant fluid from cattle," Journal of Animal Science, Vol. 73, pp. 3639- 3648 (1995). Thus, it is clear from this study that: 1) companion animals differ from humans in their ability to metabolize and utilize dietary fibers including fructooligosaccharide; and 2) that biological effects of dietary fibers will vary between dogs, cats and humans based on these differences, and that studies utilizing humans will not necessarily be predictive of findings in companion animals such as dogs and cats.

E.A. Flickinger et. al. J. Anim. Sci. 2003, 81, 2008-2018, relates to a study of nutrient digestabilities, micriobial populations and protein catabolities as effected by fructan supplementation of dog diets.

L.N. Twomey et. al. Anim. Feed Sci. and Tech. 2003, 108, 83-93, relates to a study of effects of added fructooligosaccharide and inulinase on faecal quality and digestability in dogs.

M.D. Howard et. al. Nut. Res. Vol 20, no 10, 1473-1484, relates to a study of source of dietary fiber fed to dogs affects nitrogen and energy metabolism and intestinal microflora populations.

M. Diez et. al. Res. Vet. Sci. 1998, 64, 91-96, relates to a study of influence of sugar-beet fibre, guar gum and inulin on nutrient digestability, water consumption and plasma metabolites in healthy beagle dogs.

S. Hussein et. al. J. Nut. Sci. 1999, 1454-1456, relates to a study of petfood applications of inulin and oligofrutose.

M.D. Willard et. al. Am. J. vet. Res. Vol 55, no 5, 1994, 654-658, relates to a study of effects of dietary supplementation of fructo-oligosaccharides on small intestinal bacterial overgrowth in dogs.

J. Zentek et. al. J. Anim. Phys. A. Anim. Nutr. 87, 2003, 397-407, relates to a study of dietary effects on bifidobacteria and clostrium perfringens in the canine intestinal tract.

US5776524 relates to a petfood products which are useful in reducing the amount of harmful bacteria in the small intestine.

US5952033 relates to gelatinized cereal pet food comprising a plant material, which is source of inulin.

US6596332 relates to gelatinized cereal pet food comprising a plant material, which is source of inulin, for example chicory.

WO99/22604 relates to a pet food product which comprises chicory in an amount which maintains good faeces quality or improves faeces quality of a pet and/or maintains good gastrointestinal tract health or improves the gastrointestinal health of a pet.

It has been surprisingly discovered herein that companion animals utilize fermentable fiber in a unique manner, for example relative to nutrient digestibility and calcium absorption, thereby leading to novel therapies for treating gastrointestinal health, improving bone health, and other treatments. Even further, based on the research of the present inventors, previous work related to

companion animals such as dogs may have been incomplete in terms of the beneficial effects of fermentable fiber relative to nutrient digestibility and related action. For example, using small numbers of dogs, previous work has indicated that total tract nutrient digestibility is not affected through administration of fermentable fiber in the dog. See e.g., Flickinger et al., "Nutrient digestibilities, microbial populations, and protein catabolites as affected by fructan supplementation of dog diets," J. Anim. Sci., Vol. 81, pp. 2008 - 2018 (2003). However, surprisingly, based on the further study by the inventors, action such as nutrient digestibility has now been shown to be affected through use of fermentable fiber in the companion animal, including the dog. This and other benefits of the present invention are described herein.

## SUMMARY OF THE INVENTION

The present invention relates to an oral composition comprising *0.01% to 0.15%* fructooligosaccharide, by weight of the composition, wherein said fructooligosaccharide is a short chain oligofructose, comprising from 30% to 40% 1-kestose, from 50% to 60% nystose and from 5% to 15% 1F-beta-fructofuranosylnustose, by weight of the short chain oligofructose and wherein said oral composition is a nutritionally balanced food for in use in method for treatment to enhance total tract digestibility of one or more dietary components in a companion animal.

## DETAILED DESCRIPTION OF THE INVENTION

All percentages and ratios are calculated by weight unless otherwise indicated. All percentages and ratios are calculated based on the total composition unless otherwise indicated.

Referenced herein are trade names for components including various ingredients utilized in the present invention. The inventors herein do not intend to be limited by materials under a certain trade name. Equivalent materials (e.g., those obtained from a different source under a different name or reference number) to those referenced by trade name may be substituted and utilized in the descriptions herein.

In the description of the invention various embodiments and/or individual components are disclosed. As will be apparent to the ordinarily skilled practitioner, all combinations of such embodiments and components are possible and can result in preferred executions of the present invention.

The compositions herein may comprise, consist essentially of, or consist of any of the components as described herein.

*Methods and Kits of the Present Invention*

The presently inventive methods and kits utilize companion animal compositions comprising a fructooligosaccharide. Fructooliogosaccharides are naturally occuring compounds which can be found in a variety of fruits or vegetables including banana, barley, garlic, honey, onion, rye, brown sugar, tomato, asparagus, artichoke, wheat, yacon, or chicory. Fructooligosaccharide may for example be provided as chicory, as inulin, or as short chain oligofructose. Particularly useful herein are fructooligosaccharide comprising at least one of 1-kestose (abbreviated as $GF_2$), nystose ($GF_3$), and 1F-beta-fructofuranosylnystose ($GF_4$). While fructooligosaccharides can be extracted from plants such as those mentioned herein, they can also be formed artificially by adding one, two, or three fructose, units to a sucrose molecule by a B-(2-1)-glycosidic linkage of the fructose unit(s) to the fructose unit of sucrose. As an example, fructooligosaccharides are commercially available under the tradename NUTRAFLORA from Golden Technologies Company, Incorporated (which is a short chain oligofructose comprising 1-kestose, nystose, and 1F-beta-fructofuranosylnystose. As another example, a mixture of short chain fructooligosaccharide and inulin can be PREBIO1 or a mixture of commercially available RAFTILOSE and RAFTILINE.

The fructooligosaccharide may be a short chain oligofructose, which will be well-known to those of ordinary skill in the art. Particularly useful herein are short chain Oligofructose comprising 1-kestose (abbreviated as $GF_2$), nystose ($GF_3$), and 1F-beta-fructofuranosylnystose ($GF_4$). In a preferred embodiment, the short chain oligofructose comprises from 25% to 45% 1-kestose, from 25% to 45% nystose, and from 1% to 20% 1F-beta-fructofuranosylnystose, by weight of the short chain oligofructose, alternatively from 30% to 40% 1-kestose, from 50% to 60% nystose, and from 5% to 15% 1F-beta-fructofutanosylnystose, by weight of the short chain oligofructose. As an example, short chain oligofructose is commercially available under the tradename NUTRAFLORA from Golden Technologies Company, Incorporated (which is a short chain Oligofructose comprising 35% 1-kestose, 55% nystose, and 10% 1F-beta-fructofuranosylnystose, all by weight of the short chain oligofructose).

In an embodiment herein, the fructooligosaccharide may display certain organic matter disappearance percentages. In this optional embodiment, the fructooligosaccharide may have an organic matter disappearance (OMD) of from 15% to 60% when fermented by fecal bacteria *in vitro* over a 24 hour period. That is, from 15% to 50% of the total organic matter originally present is fermented and converted by the fecal bacteria. The organic matter disappearance of the fructooligosaccharide is alternatively from 20% to 50%, alternatively from 30% to 40%.

Thus, *in vitro* OMD percentage may be calculated as follows:

$$(1-((OM\ residue-OM\ blank)\ /\ original\ OM))\ x\ 100$$

where OM residue is the organic matter recovered after 24 hours of fermentation, OM blank is the organic matter recovered in corresponding blank tubes (i.e., tubes containing medium and diluted feces, but no substrate), and original OM is that organic matter placed into the tube prior to fermentation. Additional details of the procedure are found in Sunvold et al., J. Anim. Sci., Vol. 73, pp. 1099-1109 (1995).

The companion animal compositions may comprise various levels of fructooligosaccharide, as described herein. For example, the compositions may comprise from from 0.01% to 0.15% of fructooligosaccharide, all by weight of the companion animal composition.

As stated, the fructooligosaccharide utilized herein may be chicory or inulin. Alternatively, in an optional embodiment herein, the compositions are substantially free of inulin and/or chicory (also commonly referenced as chicory root). As used herein, "substantially free of," with reference to the material, means that the composition comprises less than 0.1% of the referenced material, more preferably less than 0.05% of the referenced material, even more preferably less than 0.01% of the referenced material, even more preferably less than 0.005% of the referenced material, all by weight of the composition.

Optionally, the composition herein may be a food composition such as a dry composition (for example, kibble), semi-moist composition, wet composition, or any mixture thereof. Alternatively or additionally, the composition is a supplement, such as a gravy, drinking water, yogurt, powder, suspension, chews, treats (e.g., biscuits) or any other delivery form.

Moreover, in a preferred embodiment the composition is nutritionally balanced. As used herein, the term "nutritionally balanced," with reference to the companion animal composition, means that the composition has known required nutrients to sustain life in proper amounts and proportion based on recommendations of recognized authorities in the field of companion animal nutrition.

The compositions herein may optionally comprise one or more further components. Other components are beneficial for inclusion in the compositions used herein, but are optional for purposes of the invention. For example, as stated, food compositions are preferably nutritionally balanced. In one embodiment, the food compositions may comprise, on a dry matter basis, from 20% to 50% crude protein, alternatively from 20% to 40% crude protein, by weight of the food composition, or alternatively from 20% to 35% crude protein. The crude protein material may comprise vegetable proteins such as soybean, cottonseed, and peanut, or animal proteins such as casein, albumin, and meat protein. Non-limiting examples of meat protein useful herein include a protein source selected from the group consisting of beef, pork, lamb, poultry, fish, vegetable, and mixtures thereof.

Furthermore, the compositions may comprise, on a dry matter basis, from 5% to 40% fat, alternatively from 10% to 35% fat, by weight of the food composition.

The compositions of the present invention may further comprise a source of carbohydrate. Grains or cereals such as rice, corn, milo, sorghum, barley, alfalfa, wheat, and the like are illustrative sources.

The compositions may also contain other materials such as dried whey and other dairy by products.

The compositions may further comprise a fiber source additional to the short chain oligofructose. A variety of soluble or insoluble fibers may be utilized, which will be well-known to those of ordinary skill in the art. In one embodiment, at least a portion of the fiber source is selected from beet pulp (from sugar beet), gum arabic, gum talha, psyllium, rice bran, carob bean gum, citrus pulp, pectin, fructooligosaccharide, mannanoligofructose, soy fiber, arabinogalactan, galactooligosaccharide, arabinoxylan, and mixtures thereof.

In one embodiment, the additional fiber source comprises a fermentable fiber. Fermentable fibers are not digested by mammals but may be metabolized by intestinal bacterial species, such as Bifidobacterium. However, not all intestinal bacteria can metabolize fermentable fiber. In particular, bacteria such as Salmonella, *E. coli* and Clostridia are unable to process such fiber to any meaningful degree. This preferential digestibility, which is applicable for fermentable fiber as a class, can be used to improve the overall bacterial flora in the small intestine of the companion animal. Because fermentable fibers will only feed "good" bacteria such as Lactobacillus and Bifidobacterium, the amounts of harmful bacteria such as Salmonella, *E. coli* and Clostridia may decrease due to a reduction in food resources. Therefore, by providing a preferred food source for beneficial bacterial species, a diet supplemented with fermentable fiber can increase "good" intestinal bacteria while reducing the amount of "bad" bacteria.

The compositions may optionally comprise a total dietary fiber level of from 0.001% to 30%, alternatively from 0.01% to 20%, or alternatively from 1% to 16% total dietary fiber, by weight of the composition.

The compositions may also comprise one or more nutrients. Consistent with the benefits associated with enhanced calcium absorption herein, one of ordinary skill may optionally lower the levels of calcium relative to that which is present in typical, or premium, companion animal compositions. For example, optionally, the compositions comprise less than about 1% calcium, by weight of the composition, alternatively from 0.01% to 0.95% calcium, alternatively from 0.1% to 0.95% calcium, alternatively from 0.5% to 0.95% calcium, all by weight of the composition.

*Methods of the Present Invention*

The methods of the present invention comprise administering (*i.e.*, through ingestion) a composition of the present invention to a companion animal to provide one or more of the health benefits described herein.

In one embodiment, the invention relates to methods of enhancing total tract digestibility of one or more dietary components in a companion animal. Total tract digestibility is well-known in the art, and methods of determining total tract digestibility, including total tract ash digestibility, total tract fiber digestibility, total tract fat digestibility, and total tract dry matter digestibility are described herein below. The dietary component may be any dietary component which the ordinarily skilled artisan desires for enhancement, examples of which may be a nutrient or other component of food. In one embodiment, the method relates to enhancing total tract digestibility of a plurality of dietary components.

Generally, enhancement of calcium absorption is well-known in the art. Moreover, bone health benefits include, but are not limited to, preventing, inhibiting, ceasing, and / or reversing bone loss and / or building bone mass, and / or preventing, inhibiting, ceasing, and / or reversing osteoporosis. Moreover bone health benefits include preventing, treating and / or reducing the severity of any one of a number of bone disorders specific to companion animals (hip dysplasia, osteoarthritis, mobility issues, and the like). Thus, improved bone health may provide, for example, healthy bones, stronger bones, and / or increased bone mass. See e.g., Ohta et al., "A Combination of Dietary Fructooligosaccharides and Isoflavone Conjugates Increases Femoral Bone Mineral Density and Equol Production in Ovariectomized Mice," The Journal of Nutrition, July 2002, pp. 2048 - 2053; Mineo et al., "Various Indigestible Saccharides Enhance Net Calcium Transport from the Epithelium of the Small and Large Intestine of Rats In Vitro," The Journal of Nutrition, Dec. 2001, pp. 3243 - 3246; Takahara et al., "Fructooligosaccharide Consumption Enhances Femoral Bone Volume and Mineral Concentrations in Rats," The Journal of Nutrition, July 2000, pp. 1792 - 1795; Morohashi et al., "True Calcium Absorption in the Intestine is Enhanced by Fructooligosaccharide Feeding in Rats," The Journal of Nutrition, Oct. 1998, pp. 1815 - 1818.

The inventors herein have further discovered the importance of the fructooligosaccharide component described herein relative to strength and physical activity performance of a companion animal. Without intending to be limited by theory, it is believed that the enhanced calcium absorption herein demonstrated with respect to the companion animal upon ingestion of the composition comprising fructooligosaccharide is a precursor to enhancements in both strength and physical activity performance. Indeed, this is supported by known activity of calcium relative to muscle contraction and related mechanisms. Strength and physical performance are described in a variety of diverse fields of art, and is readily measurable by various techniques and ready experimentation. See e.g., U.S. Patent Application Publication 2003/0194478 and U.S. Patent No. 6,117,872.

As used herein, the term "administering" with respect to the companion animal means that the animal ingests or a human is directed to feed, or does feed, the animal one or more compositions herein. Wherein the human is directed to feed the composition, such direction may be that which instructs and / or informs the human that use of the composition may and / or will provide the referenced benefit, for example, enhancing total tract digestibility. For example, such direction may be oral direction (*e.g.*, through oral instruction from, for example, a veterinarian or other health professional), radio or television media (*i.e.*, advertisement), or written direction (*e.g.*, through written direction from, for example, a veterinarian or other health professional (*e.g.*, scripts), sales professional or organization (*e.g.*, through, for example, marketing brochures, pamphlets, or other instructive paraphernalia), written media (*e.g.*, internet, electronic mail, or other computer-related media)), and / or packaging associated with the composition (*e.g.*, a label present on a container holding the composition). As used herein, "written" means through words, pictures, symbols, and / or other visible descriptors. Such information need not utilize the actual words used herein, for example, "total", "tract", or "absorption", but rather use of words, pictures, symbols, and the like conveying the same or similar meaning are contemplated within the scope of this invention.

The compositions described herein may be used as a supplement to ordinary dietetic requirements or may serve as the primary food for the companion animal (and, as such, the supplements or foods may be nutritionally balanced). Administration may be on as as-needed or as-desired basis, for example, once-monthly, once-weekly, or daily (including multiple times daily). When utilized as a supplement to ordinary dietetic requirements, the composition may be administered directly to the companion animal or otherwise contacted with or admixed with companion animal food. When utilized as a companion animal food, administration will be well-known to those of ordinary skill. The amount of composition utilized may be dependent on a variety of factors, including the quality of gastrointestinal health of the animal, preference of the animal as determined by the guardian of the animal or other person administering the composition, the quality of the companion animal food, and size or breed or the companion animal.

*Methods of Analysis*

Various methods may be utilized to determine the action fructooligosaccharide will have in relation to a given method of use. Non-limiting examples follow:

Methods of Determining Tract Digestibility

Methods of analysis for determining tract digestibility of one or more dietary components will be well-known to those of ordinary skill in the art. As a non-limiting example, the following methods may be utilized in such regard:

*Animals and Diets.* In a crossover design, healthy adult dogs (n=56) are randomly assigned to 1 of 2 groups: Group 1, 21 days of feeding a diet containing 0% fructooligosacharride (0% FOS) followed by 28 days of feeding a diet containing 0.15% fructooligosaccharide (0.15% FOS): and Group 2, 21 days of feeding a diet containing 0% FOS followed by 28 days of feeding a diet containing 0.25% fructooligosaccharide (0.25% FOS). Dogs are provided meals and water at 2 time points during the day and the uneaten food and water is recorded.

*Sampling Procedures.* Procedures for this experiment are conducted under a research protocol which is approved for the protection of the health and well-being of animals. Each period of the study includes an adaptation phase (16 day for 0% FOS and 23 day for 0.15% and 0.25% FOS) followed by a five-day total excreta collection phase. During the adaptation phase food amounts are adjusted to maintain weight balance. During the five-day collection period, dogs are housed in stainless steel metabolism cages and all cages are equipped with a wire-mesh floor and urine drip pan to allow separation of feces and urine. During the entire experiment, dogs are in a temperature controlled room (20 °C), with a 15-hour dark / 9-hour light cycle. Dietary metabolizable energy (ME) values and calculated ME requirements (based on weight changes) are used to establish daily food allowances for the collection phase. All dogs are offered one-half of their daily allowance each day at 0645 and 1200, and any feed refusals are collected, weighed, and discarded. Each dog is offered 1600 mL of water each day. Dogs are weighed weekly during the experiment

Urine samples are collected in vessels containing 20 mL of 6 N HCL to prevent microbial growth and ammonia volatilization. The daily urine output is measured, saved, and composited for subsequent laboratory analyses. Feces are collected, weighed, and scored for consistency daily, with 100 g saved and composited. Composited fecal samples are lyophilized for 3-d, ground (2-mm screen; Wiley mill), and subsequently analyzed to determine nutrient digestibility.

*Chemical Analyses.* Feed and fecal samples are analyzed for dry matter (DM), ash, and nitrogen (N) (Association of Official Analytical Chemist Official Method, 1995). Fat content of feed (2 g) and feces (1 g) is determined using acid hydrolysis (Association of Official Analytical Chemist Official Method, 1995) for 45 minutes at 75.5 °C in 25% HCL (10 mL) followed by ether extraction (120 mL). Total dietary fiber (TDF) concentrations in the feed and fecal samples are determined by the AOAC method. Gross energy determinations of feed, fecal, and urine samples are conducted using a bomb calorimeter (Parr Instrument, Moline, IL). Protein digestibility (%) is calculated on a crude protein (CP) basis. Total tract digestibility is calculated using the following formulas:

$$\text{Total Tract Digestibility} = \{(\text{dietary intake} - \text{total fecal nutrient output}) / \text{dietary intake}\} \times 100\%$$

Calcium is measured in diet, feces and urine using inductively coupled plasma mass spectrometry.
Calcium retention is determined using the following formula:

$$\text{Calcium Absorption} = (\text{dietary calcium intake} - (\text{fecal calcium} + \text{urine calcium}))$$

Urine samples are prepared for energy determination by drying a 3 to 4 g sample at 105 °C in a forced-air oven. The dried samples are combined with a benzoic acid tablet prior to combustion. The GE value of each sample is corrected for the addition of benzoic acid (AOAC, 1995).

*Methods of Making*

The presently described companion animal compositions are made according to methods which will be well known by the ordinarily skilled artisan. To illustrate, the compositions of the present invention may be prepared by mixing all components singularly or in suitable combinations together, and in water where appropriate, agitating mechanically until all of the ingredients have been solubilized, dispersed, or otherwise mixed, as applicable. Wherein certain processes such as extrusion (to form kibbles, for example) are utilized, such processes will be well-known in the art.

*Examples*

The following are non-limiting examples of the present compositions which are prepared utilizing conventional methods. The following examples are provided to illustrate the invention and are not intended to limit the scope thereof in any manner, which is determined by the claims.

Example 1

Two kibble compositions having the following components at the approximate indicated amounts are prepared using methods which are standard in the art and are fed to dogs, each resulting in improved gastrointestinal health and improved fecal odor:

| Component | Example 1A (Component Amount indicated as Wt%) | Example 1B (Component Amount indicated as Wt%) |
|---|---|---|
| Short Chain Oligofructose (NUTRAFLORA, commercially available from Golden Technologies Company, Incorporated | 0.19 | 0.15 |
| Poultry, Poultry By-product Meal, and Fish Meal | 44 | 47 |
| Animal Fat | 8 | 6 |
| Beet Pulp | 2 | 3 |
| Salts | 2.5 | 2 |
| Vitamins and Minerals* | 1 | 1 |
| Minors | 3.5 | 4 |
| Grains (corn, sorghum, barley, rice) | Remainder | Remainder |
| *Vitamins and Minerals include: Vitamin E, beta-carotene and Vitamin A, Zinc Oxide, Ascorbic Acid, Manganese Sulfate, Copper Sulfate, Manganous Oxide, Calcium Pantothenate, Biotin, Vitamin $B_{12}$, Vitamin $B_1$, Niacin, Vitamin $B_2$, Vitamin $B_6$, Vitamin $D_3$, Folic Acid. | | |

Example 2

Total tract digestibility following administration of the kibble foods of Example 1 are each tested in the dog. As the samples tested are foods, the foods each contain a plurality of dietary components. The test is conducted in accordance with the descriptions provided herein above with respect to total tract digestibility, and specifically ash digestibility, fiber digestibility, fat digestibility, and dry matter digestibility. Trending increases in each of ash digestibility, fiber digestibility and dry matter digestibility are shown with the kibble food of Example 1A, with significant increases in fat digestibility with this kibble food. Significant increases in each of ash digestibility, fiber digestibility, fat digestibility, and dry matter digestibility are shown with the kibble food of Example 1B. These results are unexpected relative to the art.

Example 3

Calcium absorption following administration of the kibble foods of Example 1 is tested in the dog. As the samples tested are foods, the foods each contain a plurality of dietary components. The test is conducted in accordance with the descriptions provided herein above with respect to calcium absorption. Significant increases in calcium absorption are shown with the kibble foods of both Example 1A and Example 1B. Moreover, following administration of the kibble foods of Example 1, the dogs exhibit increased strength and physical performance as observed according to a variety of known models. These results are unexpected relative to the art.

**Claims**

1.   The oral composition comprising *0.01% to 0,15%* fructooligosaccharide, by weight of the composition, wherein said fructooligosaccharide is a short chain oligofructose, comprising from 30% to 40% 1-kestose, from 50% to 60% nystose and from 5% to 15% 1F-beta-fructofuranosylnustose, by weight of the short chain oligofructose and wherein said oral composition is a nutritionally balanced food for in use in method for treatment to enhance total tract digestibility of one or more dietary components in a companion animal.

**EP 1 696 734 B1**

**2.** The oral composition according to claim 1, wherein said enhanced total tract digestibility is selected from the group consisting of total tract ash digestibility, total tract fiber digestibility, total tract fat digestibility, total tract dry matter digestibility and compositions thereof.

**3.** The oral composition according to any of the preceding claims, wherein the oral composition further comprises a protein source selected from beef, pork, lamb, poultry, fish, vegetable, and mixtures thereof.

**4.** The oral composition according to any of the preceding claims, wherein the companion animal is a cat or a dog.

**Patentansprüche**

**1.** Mundpflegezusammensetzung, die zu 0,01 Gew.-% bis 0,15 Gew.-% der Zusammensetzung Fructooligosaccharid umfasst, wobei das Fructooligosaccharid eine kurzkettige Oligofructose ist, die zu 30 Gew.-% bis 40 Gew.-% der kurzkettigen Oligofructose 1-Kestose, zu 50 Gew.-% bis 60 Gew.-% der kurzkettigen Oligofructose Nystose und zu 5 Gew.-% bis 15 Gew.-% der kurzkettigen Oligofructose 1F-beta-Fructofuranosylnustose umfasst, und wobei die Mundpflegezusammensetzung ein ernährungsphysiologisch ausgewogenes Nahrungsmittel zum Gebrauch in Behandlungsverfahren zur Verbesserung der Gesamtverdaulichkeit im Magen-Darm-Trakt von einem oder mehreren Bestandteilen bei einem Haustier ist.

**2.** Mundpflegezusammensetzung nach Anspruch 1, wobei die verbesserte insgesamte Verdaulichkeit im Magen-Darm-Trakt ausgewählt ist aus der Gruppe bestehend aus Rohasche-Gesamtverdaulichkeit, Faser-Gesamtverdaulichkeit, Fett-Gesamtverdaulichkeit und Trockenmasse-Gesamtverdaulichkeit im Magen-Darm-Trakt sowie Zusammensetzungen davon.

**3.** Mundpflegezusammensetzung nach einem der vorstehenden Ansprüche, wobei die Mundpflegezusammensetzung ferner eine Proteinquelle ausgewählt aus Rindfleisch, Schweinefleisch, Lammfleisch, Geflügelfleisch, Fisch, Gemüse und Mischungen davon umfasst.

**4.** Mundpflegezusammensetzung nach einem der vorstehenden Ansprüche, wobei das Haustier eine Katze oder ein Hund ist.

**Revendications**

**1.** Composition orale comprenant de 0,01 % à 0,15 % en poids de la composition en fructooligosaccharide, dans laquelle le fructooligosaccharide est un oligofructose à chaîne courte, comprenant de 30 % à 40 % de 1-kestose, de 50 % à 60 % de nystose et de 5 % à 15 % de 1F-beta-fructofuranosylnustose, en poids de l'oligofructose à chaîne courte et ladite composition orale étant un aliment équilibré sur le plan nutritionnel utilisé dans un procédé de traitement destiné à améliorer la digestibilité totale dans le tractus d'un ou de plusieurs composants diététiques chez un animal de compagnie.

**2.** Composition orale selon la revendication 1, dans laquelle ladite digestibilité totale améliorée dans le tractus est choisie dans le groupe comprenant la digestibilité totale des cendres dans le tractus, la digestibilité totale des fibres dans le tractus, la digestibilité totale des fibres dans le tractus, la digestibilité totale des matières sèches dans le tractus et des compositions comprenant celle-ci.

**3.** Composition orale selon l'une quelconque des revendications précédentes, la composition orale comprenant en outre une source de protéines choisie parmi le boeuf, le porc, le mouton, la volaille, le poisson, les légumes, et des mélanges de ceux-ci.

**4.** Composition orale selon l'une quelconque des revendications précédentes, dans laquelle l'animal de compagnie est un chat ou un chien.

## REFERENCES CITED IN THE DESCRIPTION

**Patent documents cited in the description**

- US 5776524 A
- US 5952033 A
- US 6596332 B
- WO 9922604 A
- US 20030194478 A
- US 6117872 A

**Non-patent literature cited in the description**

- **SUNVOLD et al.** In vitro fermentation of cellulose, beet pulp, citrus pulp, and citrus pectin using fecal inoculum from cats, dogs, horses, and pigs and ruminant fluid from cattle. *Journal of Animal Science,* 1995, vol. 73, 3639-3648
- **E.A. FLICKINGER.** *J. Anim. Sci.,* 2003, vol. 81, 2008-2018
- **L.N. TWOMEY.** *Anim. Feed Sci. and Tech.,* 2003, vol. 108, 83-93
- **M.D. HOWARD.** *Nut. Res.,* vol. 20 (10), 1473-1484
- **M. DIEZ.** *Res. Vet. Sci.,* 1998, vol. 64, 91-96
- **S. HUSSEIN.** *J. Nut. Sci.,* 1999, 1454-1456
- **M.D. WILLARD.** *Am. J. vet. Res.,* 1994, vol. 55 (5), 654-658
- **J. ZENTEK.** *J. Anim. Phys. A. Anim. Nutr.,* 2003, vol. 87, 397-407
- **FLICKINGER et al.** Nutrient digestibilities, microbial populations, and protein catabolites as affected by fructan supplementation of dog diets. *J. Anim. Sci.,* 2003, vol. 81, 2008-2018
- **SUNVOLD et al.** *J. Anim. Sci.,* 1995, vol. 73, 1099-1109
- **OHTA et al.** A Combination of Dietary Fructooligosaccharides and Isoflavone Conjugates Increases Femoral Bone Mineral Density and Equol Production in Ovariectomized Mice. *The Journal of Nutrition,* July 2002, 2048-2053
- **MINEO et al.** Various Indigestible Saccharides Enhance Net Calcium Transport from the Epithelium of the Small and Large Intestine of Rats In Vitro. *The Journal of Nutrition,* December 2001, 3243-3246
- **TAKAHARA et al.** Fructooligosaccharide Consumption Enhances Femoral Bone Volume and Mineral Concentrations in Rats. *The Journal of Nutrition,* July 2000, 1792-1795
- **MOROHASHI et al.** True Calcium Absorption in the Intestine is Enhanced by Fructooligosaccharide Feeding in Rats. *The Journal of Nutrition,* October 1998, 1815-1818